# EUROPEAN PATENT APPLICATION

(11) **EP 0 552 612 A2**
(43) Date of publication of application: **28.07.1993**
(21) Application number: 93100163.0
(22) Date of filing: 08.01.1993
(51) Int. Cl.: G01N 33/566, G01N 33/66, C07K 15/06

(54) **Methods for determining and isolating compounds which bind directly to nucleosolic proteins**

(30) Priority: 22.01.1992 US 823729
(71) Applicant: F. HOFFMANN-LA ROCHE AG, CH-4002 Basel (CH)
(72) Inventor: Grippo, Joseph Francis, Basking Ridge, N.J. 07920 (US); Levin, Arthur, Glen Ridge, N.J. 07028 (US)
(74) Representative: Mezger, Wolfgang, Dr.

(57) **Abstract**

Methods for determining and isolating ligands resulting from compounds which produce binding with nucleosolic proteins are described.

## Description

Nucleosolic proteins are soluble proteins localized in the nucleus of a eukcaryotic cell. Such proteins are of interest because they may mediate physiologically important events. Therefore, isolating such proteins, their natural ligands, and compounds which can interact with these proteins and ligands, is of pharmaceutical importance.

Nucleosolic proteins include DNA-binding proteins capable of regulating cell activities by binding to genetic control regions and activating or inactivating transcription of a gene. Examples of nucleosolic DNA-binding proteins are nuclear receptors. A nuclear receptor is activated by binding its ligand, which in nature is usually a specific hormone. When activated, the receptor binds to the control region of its target gene, regulating gene transcription and thereby inducing, terminating, or otherwise modifying synthesis of the gene product in response to the hormone signal.

Nuclear receptors include the steroid hormone-like receptors such as the estrogen, glucocorticoid, mineralocorticoid, progesterone, and androgen receptors. These receptors are involved in various aspects of metabolism and growth. Other nuclear receptors are the thyroid hormone receptor, the vitamin D receptor, and the retinoic acid receptors. The thyroid hormone is a major metabolic regulator. Vitamin D is implicated in the regulation of bone growth. Retinoic acid receptors are activated by the natural derivatives of vitamin A, or retinol. These derivatives are required for a wide variety of physiological events including embryonic development, maintenance of epidermal differentiation, testicular function and vision, and regulation of the proliferation and differentiation of many different cell types (1-3). Therefore, the retinoic acid receptors are of clear pharmaceutical interest. Recent evidence suggests that the activity of retinoids is mediated by interactions with a family of nuclear retinoic acid receptors that can be categorized into two subfamilies, RAR and RXR. The RAR nuclear receptors, a, β and γ bind all-trans retinoic acid (T-RA) with high affinity and alter gene expression as a consequence of this direct ligand interaction (4-10). The recently described retinoid nuclear receptor, RXRα, is activated by T-RA, yet has little affinity for binding of this ligand. The ligand binding domains of the RAR and RXR nuclear retinoid receptors are divergent, exhibiting only about 29% amino acid homology (11). In addition, recent evidence has shown that RARs and RXRs may differentially regulate particular gene sequences (12,13). Subfamilies of retinoid receptors control distinct gene pathways and thus independent sets of physiological events.

The present invention relates therefore to methods for determining and isolating ligands which bind directly to nucleosolic proteins.

Direct binding compounds may also be referred to as proximate ligands. A compound may be capable of activating a nucleosolic protein in vivo but not capable of directly binding to the protein. This is because the compound may be converted in vivo to another compound, which compound is itself the proximate ligand for the proteins, or is in its turn converted into the proximate ligand. A proximate ligand may be produced either in vivo as described above, or artificially, from another compound. It is desirable to isolate proximate ligands for nucleosolic proteins because these ligands can be used in various ways to elucidate and develop treatments for conditions implicating nucleosolic proteins. Once a proximate ligand for a nucleosolic protein is known, it can for example be used in well known assays such as competitive binding assays to discover related compounds with agonistic or with antagonistic activities.

The active ligand of any compound which binds to a nucleosolic protein can in accordance with this invention be isolated and determined. Furthermore, this invention provides a method for determining if a compound produces a ligand which binds directly to a nucleosolic protein. Therefore this invention provides a means for isolating compounds which have the activity of binding nucleosolic proteins, and also provides a screen to discover such ligands.

The following examples of nucleosolic proteins and their effects demonstrate the importance of these proteins and consequently the pharmaceutical interest of elucidating and isolating their proximate ligands. Retinoic acid receptors such as the RXR receptor are nucleosolic proteins which influence skin physiology and embryonic development. The thyroid hormone receptor is a nucleosolic protein which regulates heart function, and antagonists which inhibit the action of this receptor may be valuable antiarrhythmic drugs. The vitamin D receptor is a nucleosolic protein which affects skin and bone formation and the activity of osteoblasts. Analogs of vitamin D which activate the receptor could stimulate bone production to alleviate conditions such as type II osteoporosis, while avoiding side effects caused by vitamin D such as kidney damage. Analogs of vitamin D may also be useful in treating skin conditions such as psoriasis without vitamin D's side effects. The steroid hormone receptor family includes nucleosolic proteins in the form of nuclear receptors and receptors which are localized in the nucleus when activated. This family includes such important receptors as the estrogen, androgen, progesterone, mineralocorticoid, and glucocorticoid receptors. Agonists and antagonists of these receptors would have applications in areas as diverse as birth control, breast cancer, prostate cancer, osteoporosis, and control of inflammation. The methods disclosed herein can be used to discover novel therapeutic agents for conditions related to nucleosolic proteins.

### BRIEF DESCRIPTION OF THE FIGURES

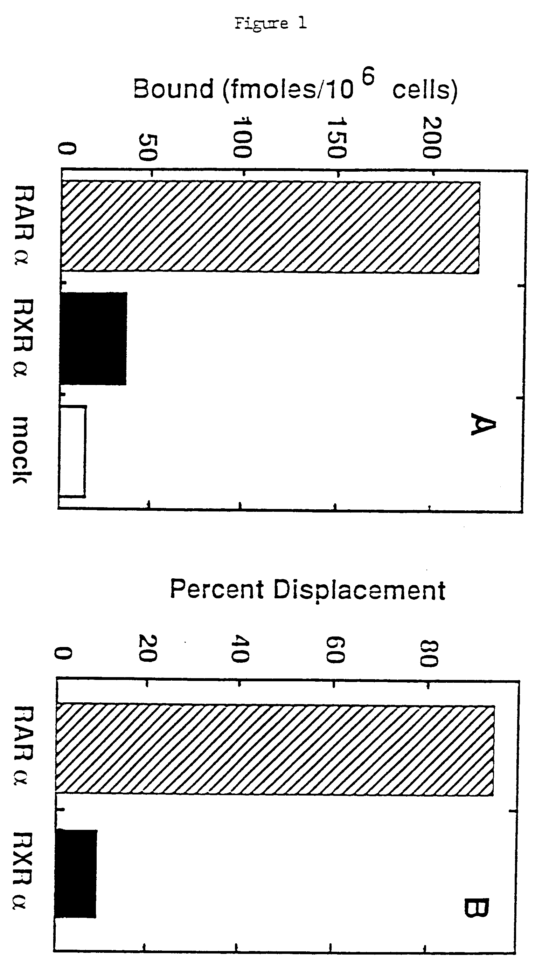

Figure 1. A, *In vitro* binding of ³H-T-RA to nucleosol preparations from RARα-, RXRα-, or mock-transfected COS-1 cells. Specific binding capacity in nucleosol fractions of COS-1 cells transfected with RARα (hatched bars), RXRα (solid bar), or mock plasmid (white bar) is expressed as fmoles/10⁶ cells. B, Displacement of tritium label from the nucleosol of transfected COS-1 cells treated in culture with ³H-T-RA. Unlabeled T-RA (10 µM) was used to displace radiolabel from the nucleosol of ³H-T-RA treated COS-1 cells transfected with RARα (hatched bar) or RXRα (solid bar). The data are presented as the percent of radiolabel displaced.

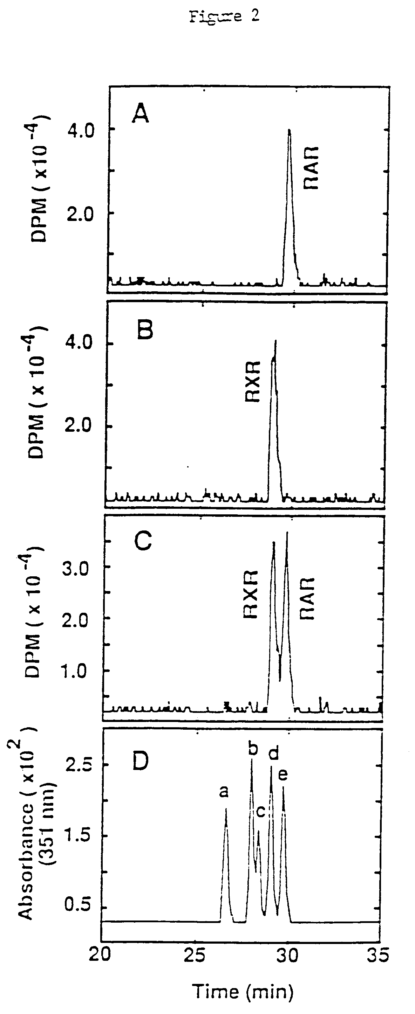

Figure 2. Reversed phase HPLC analysis of extracts of nucleosols from RARα- and RXRα-transfected COS-1 cells treated with ³H-T-RA. A, The predominant peak of radioactivity in the organic extracts of the nucleosol fractions from RARα-transfected cells elutes with a retention time of 29.8 minutes and co-elutes with authentic T-RA standard (panel D, peak e). B, The predominant peak of radioactivity in the organic extract of nucleosol fractions from RXRα-transfected cells elutes with a retention time of 29.0 minutes. This peak co-elutes with authentic standards for 9-cis RA (panel D, peak d). C, Co-injection of the organic extracts of nucleosol fractions from ³H-T-RA treated COS-1 cells transfected with either RXRα or RARα yields 2 distinct peaks of radioactivity. D, Retinoic acid isomer standards monitored at 351 nm with the following retention times (in minutes): peak a (3,4 didehydro-RA): 26.2; peak b (13-cis RA): 27.7; peak c (11-cis RA): 28.3; peak d (9-cis RA): 29.0, and peak e (all-trans RA): 29.8. Other metabolites of retinoic acid elute at the following retention times (minutes): 4-hydroxy RA: 12.8, and 4-oxo RA: 14.4.

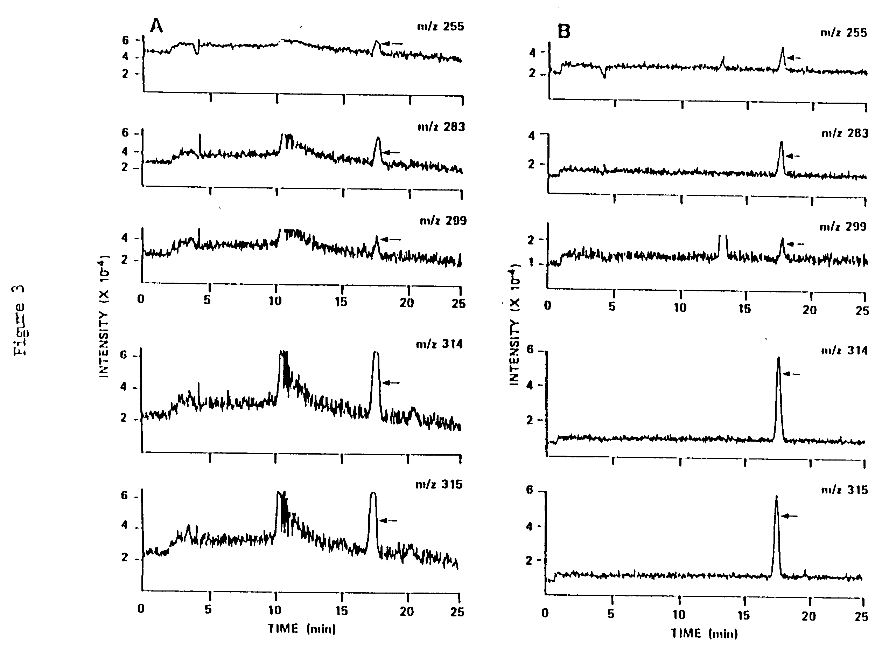

Figure 3. Qualitative identification of the RXRα extract as 9-cis RA by positive chemical ionization (PCI). The ions monitored were: m/z 315, 314, 299, 283, 255 which were assigned (M+H)⁺, (M)⁺, (M-CH₃)⁺, (M-OCH₃)⁺, (M-COOCH₃)⁺, respectively (20). A, The reconstructed selective ion current chromatograms of the methyl ester of the RXRα extract. B, The reconstructed selective ion current chromatograms of the methyl ester of authentic 9-cis RA. Analysis of the selective ion current chromatograms of the diazomethane derivative of RXRα extract yields the identical ion fragments (arrows) with the same relative ratios and retention time as the authentic methyl ester of 9-cis RA.

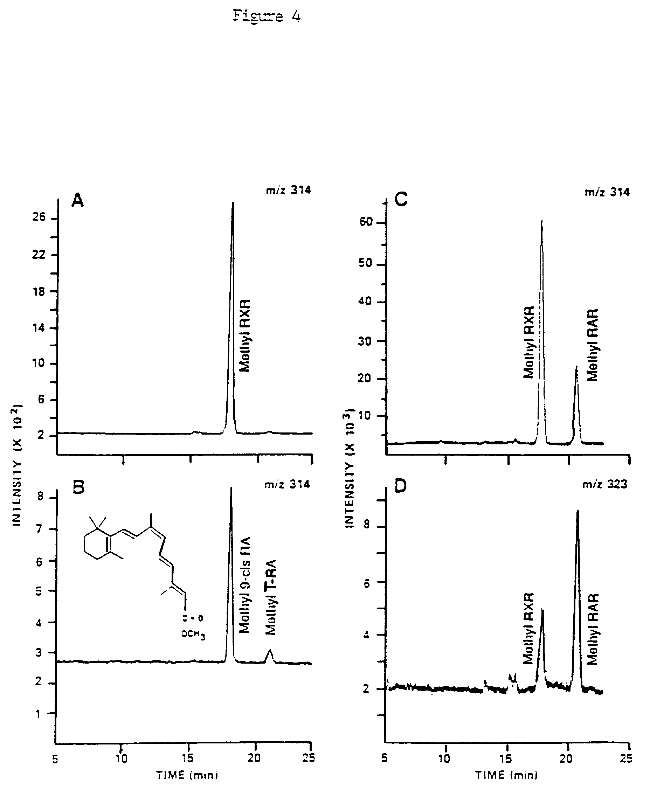

Figure 4. The NCI selective ion current chromatograms of diazomethane derivatives of nucleosol extracts and 9-cis RA standard. A,B. The diazomethane derivative of organic extracts of the trapped RXR ligand (panel A) has the same retention time (18.5 minutes) and m/z (314) as authentic methyl-9-cis RA (panel B). C,D, Co-injection of the diazomethane derivatized extracts of RXRα- and RARα- transfected COS-1 cell nucleosols treated with T-RA (panel C) or¹³C₍₉₎-TRA (panel D). Description of methods appears in the legend to Fig. 3.

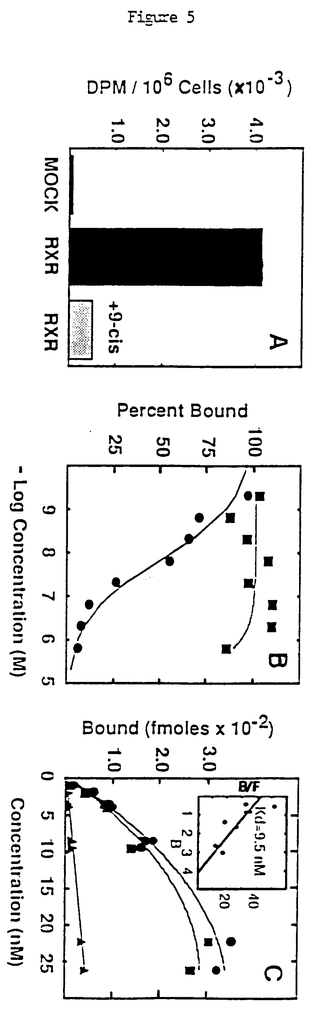

Figure 5. A, Binding of the ³H-9-cis RA(X) in COS-1 cells transfected with RXRα: or mock plasmid. ³H-9-cis RA(X) was isolated and used to treat COS-1 cells transfected with either mock plasmid (open bar) or RXRα in the absence (solid bar) or presence of a 200-fold molar excess of unlabeled 9-cis RA (crosshatched bar). B, Competition for ³H-9-cis RA(X) binding *in vitro* to RXRα-enriched nucleosol by 9-cis RA (circles) and T-RA (squares). C, Saturation kinetics and Scatchard analysis of the binding of ³H-9-cis RA(X) to RXRα. Specific binding (squares) is defined as the total binding (circles) minus the non-specific binding (triangles). Scatchard analysis (insert) of the saturation kinetics represents Bound (fmoles)/Free (nM) x 10⁶ versus Bound (fmoles).

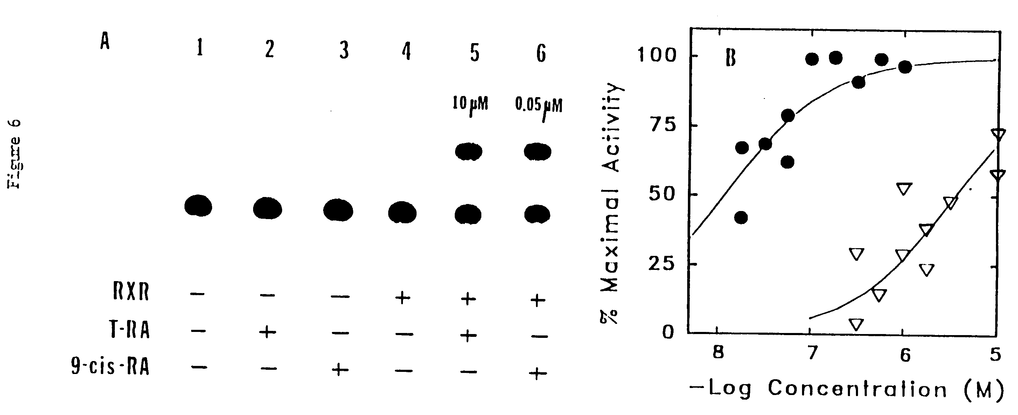

Figure 6. Comparison of 9-cis RA and T-RA induction of RXRα-dependent transcription of CRBPII-RXRE-CAT in CV-1 cells. A, CV-1 cells were co-transfected with the reporter CRBPII-RXRE-CAT construct (lanes 1-6) and either plasmid hRXRα (lanes 4-6) or control plasmid Bluescript II KS(lanes 1-3). Cells were exposed for 48 hours to either 50nM 9-cis RA (lanes 3 and 6) or 10 µM T-RA (lanes 2 and 5) or solvent (lanes 1 and 4). B, Concentration effect curves for the RXRα-dependent transactivation of CRBII-RXRE-CAT by 9-cis RA (circles) and T-RA (triangles) after 12 hours of treatment.

### DETAILED DESCRIPTION OF THE INVENTTON

The present invention provides a method for isolating the ligand of a nucleosolic protein, which ligand results from a compound which produces binding with said nucleosolic protein, i.e. a method for detecting a proximate ligand of a nucleosolic protein, which method comprises:
a. providing said compound as a detectably labelled material;
b. culturing the detectably labelled material with a eukaryotic cell expressing said nucleosolic protein
c. separating the nucleus from said eukaryotic cell, which nucleus comprises a liquid nucleosol fraction and a particulate membrane fraction; and
d. isolating the ligand from said nucleosol fraction.
An example of a nucleosolic protein is a nuclear receptor, such as a retinoic acid receptor, or a thyroid hormone receptor, or a vitamin D receptor. A particular example of a retinoic acid receptor is the RXRα receptor, whose proximate ligand is 9-cis retinoic acid or a compound which has the binding specificity of 9-cis retinoic acid. The compound itself may be the ligand. Therefore, this invention can be used for any compound which produces binding with a nucleosolic protein to determine whether the compound itself or any derivative which may be produced during the assay is the ligand which binds to the nucleosolic protein. The compound which produces binding is provided in detectably labelled form. Detectable labels and their use are well known in the art, and such labels are readily available from commercial sources. For example, isotopic labels may be used. An example of a radioactive isotopic label is tritium. An example of a nonradioactive isotopic label is ¹³C. A eukaryotic cell is transfected by methods well known in the art with expression vectors suitable for expression in said cell, which carry DNA sequences encoding a nucleosolic protein. Methods for assembling expression vectors are well known techniques of molecular biology. COS cells used in conjunction with expression vectors carrying the SV40 origin of replication are a preferred example of a readily available and suitable host-vector system. However, other hosts and vectors can also be used in this invention. After transfection, the cell is cultured by conventional methods and expression of the transfected nucleosolic protein occurs in the cell's nucleus. A preferred range of expression is about 25 to about 100-fold over the natural level of expression, or a level of expression at which the activity of any endogenous nucleosolic proteins (if any exist) would not affect the results of the assay by more than about a 5% statistical level of significance. This would represent about twentyfive to one hundred times the natural level of expression. The natural level of expression for many receptor proteins is about 1000 per cell. For example, the retinoic acid receptor is expressed at this level (14). Therefore, a preferred level of expression is about 25,000 to 100,000 receptors per transfected cell. Any desired level of expression can be attained by manipulating the transfection conditions by well known methods. The cell, which expresses the nucleosolic protein in its nucleus, is cultured with the labelled compound by conventional methods to cause the ligand resulting from the compound to bind to the nucleosolic protein in the nucleus of the cell. Any conventional method for culturing will cause the ligand resulting from the detectably labelled compound to bind to the nucleosolic protein within the nucleus. The ligand may be the compound itself or another compound resulting from transformation of the compound. Although any effective time period of culturing is contemplated, from about four to about forty-eight hours of culturing should be particularly effective. The nucleus of the cell, which now contains the bound and labelled ligand, is removed or separated from the cell by known methods (14). Any conventional method can be used to carry out the separation. The nucleus (which is made up of a membrane surrounding a fluid interior, or nucleosol) is then divided into its liquid nucleosol fraction and its particulate membrane fraction. One means of so doing is centrifugation. However, any conventional means is contemplated by this invention. In accordance with this invention it was shown that the ligand is bound to the nucleosolic protein and therefore is located in the nucleosol fraction, and may be conveniently isolated therefrom. Any conventional method, such as organic solvent extraction of the nucleosol (17) can be used to isolate the ligand, which can also be identified by any conventional means. Any labelled material which is found in the nucleosolic extract must be material that is associated with the presence of the transfected nucleosolic protein. If the nucleosolic extract has retained labelled material, then analysis of the labelled material will determine the identity of the proximate ligand for the transfected nucleosolic protein. Methods for identifying and characterizing chemical compounds are well known in the art. Various conventional chromatography techniques may be used, for cxample, high pressure liquid chromatography (HPLC). Other techniques include mass spectrophotometry (GC/MS, LC/MS, and NCI-MS), NMR, and in the case of peptides, amino acid sequencing.

The present invention also includes a method for determining whether a compound produces a ligand which binds directly to a nucleosolic protein. The principles and methods which apply to the above-described isolation methods apply equally to this determination method. The method comprises culturing the compound with a eukaryotic cell transfected with an expression vector containing DNA sequences encoding the nucleosolic protein after expression of said nucleosolic protein by the cell to cause the ligand resulting from said compound to bind to the nucleosolic protein within the nucleus of the cell. The cell is then exposed to a detectably labelled compound which is known to bind to the nucleosolic protein. The nucleus containing the bound ligand is removed or separated from the cell and the nucleosolic fraction of the nucleus is examined to determine through the presence of labelled material in the nucleosolic fraction whether the ligand or the labelled compound is bound to the nucleosolic protein. If the label is present, then the labelled compound has bound. If the label is not present, then the compound or the ligand it produces has bound. This analysis is performed by methods appropriate to the detectable label selected. For example, if a radioactive label such as tritium was used in the labelled compound, then presence of label could be detected by using a scintillation counter. Methods for transfecting, culturing, labelling, isolating, and analyzing are well known in the art and are described above. The nucleosolic protein may be a receptor, such as a retinoic acid receptor, in particular the RXR receptor. The receptor may preferably be expressed at a level of about 25,000 to 100,000 receptors per cell. A possible detectable label is an isotopic label. The affinity of the nucleosolic protein for ligand may be at least about 10⁻⁸ molar. The eukaryotic cell which is transfected may be a COS cell, and the expression vector used may contain the SV40 origin of replication.

It is useful when the nucleosolic protein binds to its ligand with a sufficient affinity to maintain high specificity in these methods. A binding affinity of at least about 10⁻⁸ molar is preferable (where an affinity of 10⁻⁷ is considered lower, and an affinity of 10⁻⁹ is considered higher). Any compound which produces binding with a nucleosolic protein binds with a sufficient affinity. Also, a nucleosolic protein which acts as a nuclear receptor has sufficient affinity. For example, it is known that the steroid hormone-like receptors have binding affinities in the 10⁻⁹ or 10⁻¹⁰ range. Among the retinoic acid receptors, the RXR receptor binds 9-cis retinoic acid with an affinity of about 10⁻⁸ molar. All three of the RAR receptors (α, β and γ), bind all-trans retinoic acid (T-RA) with an affinity of about 10⁻⁹ molar (7, 14-16). The fact that nucleosolic proteins are soluble proteins found only within the nucleus is also important in that it makes high levels of purification possible, since the nucleosol can be conveniently isolated and extracted and the ligand isolated therefrom.

The examples which follow demonstrate the claimed invention, but are not intended to limit it in any way.

### Example 1

### Nucleosol Trapping of the RXR ligand

To test the hypothesis that T-RA-induced activation by RXRα is the result of conversion of T-RA to a more proximate ligand that directly binds RXRα, a method was developed to trap derivatives of T-RA bound to RXRα in whole cells. Transient transfection of COS cells with expression vectors for RARα, β, or γ yields nucleosol fracdons highly enriched for ³H-T-RA binding associated with these receptors (14-16). COS-1 cell nuclei enriched with RXRα selectively bind ligands that exhibit high affinity for RXRα. If T-RA is transformed in culture into a high affinity ligand for RXRα, then the overexpressed receptors bind this ligand. Therefore, metabolites or isomers of T-RA would be trapped in the nucleosol of RXRα transfected COS-1 cells following treatment of these cells with T-RA.

First it was determined that RXRα did not bind T-RA by conducting in *vitro* binding assays. Little binding of ³H-T-RA is detected in COS-1 cell nucleosol isolated from RXRα- or mock-transfected cells. When nucleosol fractions isolated from COS-1 cells transfected with RARα are incubated with ³H-T-RA, considerable binding is detected (Fig. 1a). Thus, the RXRα gene product expressed in the nucleosol of COS-1 cells appears to have a low affinity for T-RA binding. In contrast to the in vitro binding results presented in Fig. 1a, binding studies in whole cells show that nucleosol fractions from ³H-T-RA treated COS-1 cells transfected with either RARα or RXRα bind similar amounts of radioactivity following removal of unbound label by PD-10 size exclusion chromatography (Fig 1. legend). Binding of labeled material to nucleosol fractions is dependent on transfection with RARα or RXRα since little radiolabel is detected in the nucleosol fraction from mock-transfected cells. An excess of unlabeled T-RA displaces radioactivity bound in the RARα nucleosol, but not the tritiated material bound in the RXRα nucleosol (Fig. 1b).

COS-1 cells were grown in Dulbecco's Minimum Essential Medium supplemented with 10% heat inactivated fetal calf serum, 4 mM glutamine, kanamycin sulfate (120 mg/l), penicillin (120,000 units/ml), and streptomycin (12o µg/l). Near-confluent cells were transfected with the pSG5 expression plasmid (34) containing cDNA's for RARα, RXRα, or in the case of mock-transfected cells, a chimeric human estrogen receptor containing the DNA binding region of RARα (construct A, Ref. 35). The hRARα and hRXRα coding sequences were PCR cloned from HL60 cell mRNA and human liver mRNA (Clontech), respectively, and inserted into the expression plasmid pSG5. COS-1 cells (5x10⁷) were washed, suspended in 0.8 ml of Ca⁺⁺- and Mg⁺⁺-free phosphate buffered saline and mixed with 25 µg plasmid DNA. Transfections were performed by electroporation (Bio-Rad Genepulser) at 250 µFarads and 350 volts. Nucleosol fractions were prepared (14) 72 hours after transfection. For in vitro binding studies (panel A), aliquots of nucleosol (20 µl) were incubated for 2 hours at 4°C with 10 nM ³H-T-RA in lysis buffer as described (14). The all trans-[11,12-³H₂]-retinoic acid was obtained from NEN and checked for radiochemical purity (99.5%) by HPLC analysis. Nonspecific binding was determined in the presence of 100-fold molar excess of unlabeled T-RA. Following incubation, bound was separated from free radioactivity by eluting the incubate from Pharmacia PD10 desalting columns with 5 mM phosphate buffer, pH 7.4, containing 400 mM KCl, 10% glycerol, 1 mM dithiothreitol, and 1 mM phenylmethylsulfonyl fluoride. Bound radioactivity eluted in the column void volume and was determined by liquid scintillation counting of the eluent.

For treatment of cells in culture (panel B), COS-1 cells transfected with RARα, mock, or RXRα were treated after 24 hours with 50-200 nM ³H-T-RA and the cells were harvested 48 hours after drug treatment. The radiolabeled nucleosol fractions were obtained (14) and aliquots (containing 8,000 to 15,000 dpm of total radiolabel bound) were incubated as described above in the presence or absence (control) of 10 µM T-RA for 4 hours at 4°C. Bound activity was determined as above. Percent displacement was determined by dividing the radioactivity in the presence of T-RA by control values bound in the absence of T-RA.

### Example 2

### Extraction and analysis of trapped ligands

The failure of T-RA to displace the radiolabel from the RXRα nucleosol suggested that either the radiolabel was irreversibly bound or that the radiolabel bound in the RXRα nucleosol was not T-RA. Labeled nucleosol fractions were extracted from both RARα- and RXRα-transfected COS-1 cells with organic solvents and found that >90% of the radioactive material from RARα or RXRα nucleosol could be extracted into the organic phase. Thus most of the radiolabeled material is bound reversibly to RARα and RXRα. This result shows that a ligand distinct from T-RA was bound by RXRα in the nucleosol. HPLC analysis of the organic extracts of RARα- and RXRα-transfected nucleosol preparations indeed provided the first evidence that RXRα trapped a distinct derivative of T-RA.

The organic extracts of the RARα and RXRα nucleosols were analyzed by reversed phase HPLC to determine the identity of the receptor-bound radiolabel. A tritiated material that co-elutes with T-RA (elution time, 29.8 min.) is found in extracts from RARα-transfected cells (compare Figs. 2A and 2D, peak e). Organic extracts of nucleosol preparations from RARβ- and RARγ-transfected COS-1 cells treated with ³H-T-RA also contain predominantly ³H-T-RA. These results are consistent with in vitro data that show high affinity binding of T-RA to these receptors (7, 14-16). Remarkably, extracts from RXRα-transfected nucleosols contain very little T-RA, but contain a tritiated material that elutes earlier than T-RA (Fig. 2B, elution time, 29.0 min.). The RARα and RXRα ligands are distinct chemical entities, since baseline resolution of the two substances is achieved following co-injection of the samples (Fig. 2C). A number of polar metabolites of T-RA including 4-oxo-, 4-hydroxy- and 3,4-didehydro-, retinoic acids were tested and it was concluded that these compounds have different elution profiles than the extracted RXRα ligand (Fig. 2D).

Nucleosol fractions (0.2 to 1.0 ml) from COS-1 cells labeled in culture (Fig. 1B) were extracted with acetonitrile:butanol (1:1) (17). The organic extracts were injected with a WISP Model 712 autoinjector in volumes of 45-100 µl and eluted at a flow rate of 1.5 ml/min from a series of 2 Zorbax ODS 4.6 mm x 25 cm columns using a linear gradient of acetonitrile:20 mM ammonium acetate:acetic acid 50:50:0.5 to 95:5:0.04 in 26 minutes and holding final conditions for 12 minutes for free acids or 20 minutes for methyl esters. A flow-through radiochemical detector (IN/US β-RAM) with a 540 µl flow cell and a scintillant flow rate of 4.5 ml/min was used to determine the radioactivity in the eluent. Standard curves for converting net counts on the radiochemical detector to dpm were generated using HPLC analysis of known quantitites of ³H-T-RA. Ultraviolet absorbance was monitored at 351 nm using an Applied Biosystems 783A variable wavelength detector. Data from the radiochemical detector and the UV monitor were collected and analyzed using a Waters Maxima 820 data system. The utmost care was taken to insure that all procedures using retinoids or retinoid-treated cells or cell fractions were performed in darkness or amber light.

Transfected COS-1 cells were treated with 500 nM T-RA or 500 nM ¹³C₍₉₎-T-RA 24 hours after transfection with RARα, mock, or RXRα and harvested 48 hours after treatment. Organic extracts of the nucleosol fractions were derivatized with diazomethane (18), and fractionated by reversed phase HPLC (see legend to Fig. 2) to collect the peaks of interest. The fractions corresponding to the RXR or RAR ligands were collected, evaporated to dryness and resuspended in mobile phase. Aliquots (9 µl) were applied to a diol column (1 x 250 mm) (ES Industries) and eluted isocratically using a mobile phase of 15% toluene in n-hexane at a flow rate of 50 µl/min at 45°C. The eluent was introduced into a Finnigan 3200 mass spectrometer modified for direct liquid introduction (36) via a probe heated to 250°C. Mobile phase was used as the reagent gas for both positive chemical ionization (PCI) and negative chemical ionization (NCI).

### Example 3

### 9-cis RA is a ligand for RXRα

The reversed phase HPLC chromatographic profile of the extracted RXR ligand was characteristic of stereoisomers of T-RA. After obtaining standards for a number of natural stereoisomers of T-RA including 13-cis, 11-cis and 9-cis retinoic acids, it was found that the RXR ligand elutes with the same retention time as 9-cis RA (Compare Figs. 2B and 2D, peak d). The ligands extracted from both RARα and RXRα nucleosols were further characterized by derivatizing the organic extracts with diazomethane, thus methylating free carboxylic acid groups (18). The derivatized ligand extracted from the RARα co-elutes on the same reversed phase HPLC system shown in figure 2 with a standard for all-trans methyl retinoate (Me-T-RA, retention time, 39.5 min.), while the derivatized RXR ligand co-elutes with authentic 9-cis methyl retinoate (Me-9-cis-RA, 38.6 min.).

Microbore liquid chromatography/mass spectrometry (LC/MS) with positive and negative chemical ionization was employed to further characterize the RXR ligand (19). Using the normal phase LC/MS analytical system, derivatized RARα and RXRα ligands co-elute with Me-T-RA and Me-9-cis-RA, respectively (compare Figs. 4C and 4D). Jon fragments at the retention time of the RXRα ligand were monitored using positive chemical ionization mass spectra (PCI-MS), and compared the spectra of the methylated RXR ligand (Fig. 3A) with that of authentic Me-9-cis RA (Fig. 3B). Selective ion monitoring (SIM) of five fragment ions, which are indicative of the presence of a methyl ester of an unmodified isomer of retinoic acid (20), provided qualitative identification of the RXR ligand (see legend to Fig. 3). The methylated RXR ligand and the authentic Me-9-cis RA sample co-chromatograph and produce identical fragment ions with similar relative intensities (Fig. 3).

In addition, methylated derivatives of the RAR and RXR ligands were analyzed using negative chemical ionization mass spectrometry (NCI-MS). In this method, fragmentation is reduced and the molecular ion is generally recorded as the only peak. After monitoring the total ion current, including ions m/z 150 through 500, in the mass spectrum of the methylated RXR ligand, one ion peak is found with m/z 314. This ion peak corresponds to the molecular ion of a material coeluting with Me-9-cis-RA (Compare Fig. 4A and 4B). Thus, using NCI-MS, it was demonstrated that methylated derivatives of the RAR and RXR ligands both exhibit molecular ions at m/z 314 and co-chromatography with Me-T-RA and Me-9-cis RA, respectively.

The RXR ligand and its methyl derivative co-elute by both normal and reversed phase HPLC with 9-cis RA and methyl 9-cis RA, respectively, and the patterns of fragmentation and chemical ionization between authentic Me-9-cis RA and the methylated derivative of the RXR ligand are identical. 9-cis RA is the ligand trapped by RXRα overexpressed in COS-1 cells. Therefore, the extracted RXR ligand is referred to as 9-cis RA(X). A chemical structure of the methyl derivative of the RXR ligand, 9-cis methyl retinoate is shown in Fig. 4B.

Although 9-cis RA is a photoisomer of T-RA, it is assumed that T-RA was isomerized to 9-cis RA in COS-1 cells, since all procedures were performed in the dark or under yellow lights to prevent photoisomeriation. HPLC analysis showed that there was no 9-cis RA present in the dosing solutions at the start of each experiment. While the mechanism of isomerization is unknown, the material extracted from the RXR nucleosol is directly derived from added T-RA. When RARα- or RXRα-transfected COS1-cells were treated with ¹³C₍₉₎-T-RA, the extracted and methylated ligands trapped by RARα and RXRα co-elute with Me-T-RA and Me-9-cis RA, respectively and exhibit a molecular ion, m/z 323, consistent with the presence of nine ¹³C atoms found in the precursor T-RA (Fig. 4B).

### Example 4

### 9-cis-RA binds RXRα

³H-9-cis RA(X) binds to macromolecules in nucleosol fractions isolated from RXRα-transfected COS-1 cells. ³H-9-cis RA(X) binding to COS-1 nucleosol fractions is dependent on transfection with RXRα and is effectively inhibited by co-treatment of the COS-1 cells with a 200-fold molar excess of authentic unlabeled 9-cis RA (Fig. 5A). Direct binding of 9-cis RA(X) in the nucleosol fraction of RXRα-transfected COS-1 cells was clearly demonstrated since HPLC analysis of organic extracts of these nucleosol preparations show trapping of ³H-9-cis RA.

Direct binding of ³H-9-cis RA(X) to RXRα was further characterized in vitro using saturation kinetics, Scatchard analysis and competition assays. ³H-9-cis RA(X) exhibits saturable, high affinity binding (Kd = 9.5 nM) to RXRα in nucleosol prepared from RXRα-transfected COS-1 cells (Fig. 5C). Unlabeled 9-cis RA (IC₅₀'s 20-50 nM) but not T-RA (IC₅₀ >10 µM) competes for ³H-9-cis-RA(X) binding to RXRα nucleosol preparations. Thus, 9-cis-RA binds directly to RXRα in whole cells and in vitro and has a relatively high affinity for this receptor.

COS-1 cells transfected with RXRα or mock plasmids were treated with ³H-9-cis RA(X) 48 hours after transfection. The radioligand (³H-9-cis RA(X)) was prepared by extracting the nucleosol fractions from RXRα transfected cells treated with 200 nM ³H-T-RA. The acetonitrile/butanol extract was concentrated by evaporation under a stream of nitrogen, reconstituted in ethanol and added to COS-1 cells to yield a final concentration in the medium of 0.4 nM. After 24 hours, cells were harvested and the nucleosol fractions were analyzed to determine the bound activity as described in the legend to Fig. 1. The identity of the radiolabel in the nucleosol fractions of the ³H-9-cis RA(X)-treated cells was determined following organic extraction and HPLC analysis as described in the legend to Fig. 2.

In vitro binding studies with ³H-9-cis RA(X) were performed as described in the legend to Fig. 1. Aliquots of 10 or 15 µl of RXRα nucleosol were incubated for 6 hours at 4°C in a total volume of 50 or 100 µl for competitive binding or saturation kinetics studies, respectively. The ³H-9-cis RA(X) was concentrated by evaporation under a stream of nitrogen to form the appropriate stock solutions. In competitition binding studies, aliquots of RXRα nucleosol were incubated with 10 nM ³H-9-cis RA(X) in the presence of various concentrations of unlabeled T-RA or 9-cis RA. Saturation kinetics were performed by adding increasing concentrations (1 to 26 nM) of ³H-9 cis RA(X) to RXRα nucleosol in the presence (non-specific binding) or absence (total binding) of a 100 fold molar excess of unlabeled 9-cis RA. Bound radioligand was separated from free as described in the legend to Fig. 1. Calculations for the saturation kinetics and Scatchard analysis (37,38) were based on the specific activity of the ³H-T-RA (50.6 Ci/mmole) used in the preparation of the radiolabeled extracts. Specific activity was confirmed by LC/MS analysis showing the presence of 2 tritium atoms in the RXR radioligand.

### Example 5

### 9-cis RA Induces RXRα-Mediated Transactivation

To show that 9-cis RA can activate RXRα, a reporter plasmid (CRBPII-RXRE-CAT) with the CRBII RXR response element (12) upstream of the SV40 minimal promoter and the bacterial chloramphenicol acetyltransferase gene (CAT) was constructed. Co-transfection of CV-1 cells with RXRα and the CRBII-RXRE-CAT reporter gene results in an increase in CAT activity that is dependent on the addition of either T-RA or 9-cis RA (Fig. 6A). Similar CAT activity is obtained when CV-1 cells are treated with 0.05 µM 9-cis RA or 10.0 µM T-RA for 48 hours prior to assay (Fig. 6A). The potency of T-RA at this time point was attributed to the isomerization of T-RA to 9-cis-RA in the CV-1 cells. To minimize isomerization, CV-1 cells were treated with 9-cis RA or T-RA for only 12 hours before assaying for CAT activity. Under these conditions, there is a marked difference in the potencies of 9-cis RA and T-RA in activating CAT activity, with these compounds exhibiting EC₅₀'s (effective concentration to inhibit 50% of binding) of 18 nM and >1 µM, respectively (Fig. 6B).

To construct CRBPII-RXRE-CAT, oligos 5'-GATCTGCTGTCACAGGTCACAGGTCACAGGTCACAGTTCA-3' [SEQ ID No: 1] and 5'-GATCTGAACTGTGACCTGTGACCTGTGACCTGTGACAGCA-3' [SEQ ID No: 2] (12) were annealed and inserted into the BglII site of the vector pCAT-Promoter (Promega) upstream of the SV40 minimal promoter to generate the reporter plasmid CRBPII-RXRE-CAT. For transfection experiments, the RXRα expression plasmid (0.5 µg) (except lanes 1-3 of panel a), reporter plasmid CRBPII-RXRE-CAT (5 µg), reference plasmid pβAcLacZ (5 µg), and carrier plasmid Bluescript II KS (Stragene) were combined for a total of 15 µg and introduced into CV-1 cells (5x10⁵/100 mm plate) by calcium phosphate co-precipitation (39). Alter 18 hours, the precipitate was removed and cells were washed in phosphate buffered saline. After the initiation of transfection, various concentrations of 9-cis RA or T-RA were added to the medium. Twelve or forty-eight hours alter treatment, cells were harvested, and lysates prepared and analyzed for chloramphenicol acetyl transferase activity (CAT) (40). β-galactosidase activity was determined using Lumigal (Lumigen) according to the manufacturer's specifications. For autoradiographic presentation of the CAT activity, cell lysate volumes added to the assay tubes were normalized to β-galactosidase activity. For analyzing concentration response relationships, CAT activity was quantitated directly from TLC plates using a Betascope 603 beta analyzer (Betagen) and counts were normalized to β-galactosidase activity.

### Example 6

### 9-cis RA is preferred ligand for RXRα

Other natural derivatives of T-RA may also bind and activate RXRα. However, data from our ligand trapping experiments show that COS-1 cells overexpressing RXRα preferentially bind 9-cis RA after being exposed to a mixture of natural isomers and metabolites of retinoic acid in T-RA-treated culture medium. Additionally, RXRα-transfected COS-1 cells were exposed to photoisomerization products of ³H-T-RA. This complex ligand mixture contains at least 11 different tritiated photoisomers of T-RA, including 7-cis-, 9-cis-, 11-cis-, 13-cis- and all-trans-retinoic acids (21,22) however only 9-cis RA was trapped in the nucleosol fractions. The 9-cis RA itself does not appear to be further metabolized in COS-1 cells to another derivative that binds RXRα since 9-cis RA could be re-extracted from nucleosols of RXRα transfected COS-1 cells following treatment of the cells with this ligand. Thus under these experimental conditions the preferred ligand for RXRα is 9-cis RA.

To determine the selective Binding of a single isomerization product a stock solution (250 µl) of ³H-T-RA was obtained from NEN and UV-irradiated in a 12 x 100 mm glass culture tube with a 5.00 min exposure to >300 nm fluorescent lights. An aliquot of the resulting mixture of photoisomers was analyzed by HPLC as described in Example 2. The photoisomerized material was added to COS-1 cells transfected with RXRα. The cells were cultured for 48 hrs and the nucleosol fraction prepared. Organic extracts (17) of the nucleosol were analyzed by HPLC.

### Example 7

### T-RA is the proximate ligand for 13-cis RA

The 13-cis isomer of retinoic acid does not effectively compete in vitro with ³H-T-RA for binding to the retinoic acid receptors α, β or γ. There was minimal competition for binding even at concentrations of 13-cis RA as high as 1 µM. Under the conditions of this assay isomerization is minimized by incubating at 4°C and minimizing the incubation times. Under similar conditions unlabeled T-RA competes for binding with ³H-T-RA with EC₅₀'s (effective concentrations to inhibit 50% of binding) in the range of 5-15 nM. Curiously, 13-cis retinoic acid is an active retinoid and is used clinically in the treatment of chronic cystic acne.

Competitive binding studies were performed as follows. COS-1 cells were transfected with the cDNAs encoding RARs α, β or γ in the expression vector pSG5 (described in Example 1). The cells were cultured for 72 hours and the nucleosol fraction isolated (14). Nucleosol fractions were incubated (14) at 4°C for 30 minutes with 5 nM ³H-T-RA with various concentrations of unlabeled 13-cis RA. Bound was separated from free radioactivity using PD10 columns as described in Example 1 above.

COS-1 cells transfected with RARs α, β or γ were allowed to express the receptor protein for approximately 72 hours and then the cells were exposed to ³H-13-cis RA in the media for 4 hours prior to harvesting the cells. There was little or no ³H-T-RA found in the media alter the 4 hour incubation. However, the nucleosol fraction trapped only ³H-T-RA. Interestingly, high performace size exclusion chromatography of the labeled nucleosid fraction demonstrated that the radioactivity was associated with a 50kD fraction that could be collected for analysis of the radiolabel bound to the receptor. HPLC analysis of the organic extracts of the 50kD fraction demonstrated that the material bound was ³H-T-RA.

Ligand trap studies were performed as follows. COS-1 cells were transfected with either the RAR α, β or γ as described in Example 1 and were treated with 50 nM ³H-13-cis RA. The cells were harvested and nucleosol prepared (14) four hours alter treatment. The nucleosol fractions were extracted with organic solvents (17) and analyzed by reversed phase HPLC using the methods described in Example 2. The medium was collected and extracted (17) and the extracts analyzed by HPLC as above.

### References

1. Thaller, C. & Eichele, G., Nature **327**, 625-628 (1987).
2. Roberts, A.B. & Sporn, M.B. The Retinoids **Vol 2,** (eds. Sporn, M.B., Roberts, A.B. & Goodman, DeW.S.) 209-286 (Academic Press, Orlando, 1984).
3. Coward, W.A., Howell, J.McC., Thompson, J.N. & Pitt, G.A.J., Br.J. Nutr. **23,** 619-626 (1969).
4. Giguere, V., Ong, E.S., Segui, P. & Evans, R.M., Nature **330,** 624-629 (1987).
5. Petikovich, M., Brand, N.J., Krust, A. & Chambon, P., Nature **330,** 444-450 (1987).
6. Aström, A., Pettersson, U. Krust, A., Chambon, P. & Vorhees, J.J., Biochem. Biophys. Res. Comm. **173,** 339-345 (1990).
7. Yang, N., Schüle, R., Mangelsdorf, D.J. & Evans, R.M., Proc. Natl. Acad. Sci. USA **88,** 3559-3563 (1991).
8. Graupner, G. et al., Biochem. Biophys. Res. Comm. **179,** 1554-1561 (1991).
9. Brand, N. et al. Nature **332,** 850-853 (1988).
10. Krust, A., Kastner, Ph., Petkovich, M., Zelent, A. & Chambon, P., Proc. Natl. Acad. Sci. USA **86,** 5310-5314 (1989).
11. Mangelsdorf, D.J., Ong, E.S., Dyck, J.A. & Evans, R.M., Nature **345,** 224-229 (1990).
12. Mangelsdorf, D.J. et al., Cell **66,** 555-561 (1991).
13. Shüle, R. et al., Proc. Natl. Acad. Sci. USA **88,** 6092-6096 (1991).
14. Nervi, C.J., Grippo, J.F., Sherman, M.I., George, M.D. & Jetten, A.M. Proc. Natl. Acad. Sci. USA **86,** 5854-5858 (1989).
15. Cavey, M.T., Martin, B., Carlavan, I. & Shroot, B., Analy. Biochem. **186,** 19-23 (1990).
16. Crettaz, M., Baron, A., Siegenthaler, G. & Hunziker, W., Biochem. J. **272,** 391-397 (1990).
17. McClean, S.W., Ruddel, M.E., Gross, E.G., DeGiovanna, J.J. & Peck, G.L., Clin. Chem. **28,** 693-696 (1982).
18. De Leenheer, A.P. & Lambert, W.E. Method in Enzymology **189** (ed. Packer, L.) 104-111 (Academic Press, New York, 1990).
19. Huselton, C.A. et al. in Liquid Chromatography/Mass Spectrometry, Applications in Agricultural, Pharmaceutical and Environmental Chemistry (ed. Brown, M.A.) 166-178 (American Chemical Society, Washington, DC, 1990).
20. Napoli, J.L. in Methods in Enzymology **123** (eds. Colowick, S. and Kaplan, N.O.) 112-124 (Academic Press, NY) (1986).
21, Halley, B.A. & Nelson, E.C., J. Chromatogr. **175,** 113-123 (1979).
22. Motto, M.G. et al., J. Chromatogr. **481,** 255-262 (1989).
23. Rando, R.R., J. Bioenerg. and Biomemb. **23,** 133-146 (1991).
24. Rando, R.R. & Chang, A., J. Am. Chem. Soc. **105,** 2879-2882 (1983).
25. Ben-Amotz, A., Mokady, S. & Avron, M., Brit. J. Nutri. **59,** 443-449 (1988).
26. Ben-Amotz, Mokady, S., Edelstein, S. & Avron, M., J. Nutri. **119,** 1013-1019 (1989).
27. Evans, R.M., Science **240,** 889-895 (1988).
28. Glass, C.K., Devary, O.V. & Rosenfeld, M.G., Cell **63,** 729-738 (1990).
29. Glass, C.K., Lipkin, S.M., Devary, O.V. & Rosenfeld, M.G., Cell **59,** 697-708 (1989).
30. Husmann, M., Lehmann, J., Hoffmann, B., Hermann, T., Tzukerman, M. & Pfahl, M., Molec. Cell. Biol. **11,** 4097-4103 (1991).
31. Hudson, L.G., Santon, J.B., Glass, C.K. & Gill, G.N., Cell **62,** 1165-1175 (1990).
32, Zhang, X.K. et al., New Biol. **2,** 169-181 (1991).
33. Graupner, G., Wills, K.N., Tzukerman, M., Zhang, X.-K. & Pfahl, M., Nature **340,** 653-656 (1989).
34. Green, S., Isseman, I. & Scheer, E., Nucleic Acids Res. **16,** 369 (1988).
35. Dalman, F.C. et al., Biochemistry **30,** 5605-5608 (1991).
36. Garland, W.A. et al., Trends in Analy. Chem. **3,** 177-184 (1991).
37. Scatchard, G., Ann. N.Y. Acad. Sci. **51,** 660-672 (1949).
38. Grippo, J.F. & Gudas, L.J., J. Biol. Chem. **262,** 4492-4500 (1987).
39. Chen, C. & Okayama, H., Molec. Cell. Biol. **7,** 2745-2752 (1987).
40. Rosenthal, N., Methods in Enzymology **152,** (eds. Berger, S.L. & Kimmel, A.R.) 704-720 (Academic Press, NY, 1987).

## Claims

1. A method for isolating the ligand of a nucleosolic protein, which ligand results from a compound which produces binding with said nucleosolic protein which comprises:
a. providing said compound as a detectably labelled material;
b. culturing the detectably labelled material with a eukaryotic cell expressing said nucleosolic protein
c. separating the nucleus from said eukaryotic cell, which nucleus comprises a liquid nucleosol fraction and a particulate membrane fraction; and
d. isolating the ligand from said nucleosol fraction.

2. A method for determining whether a compound produces a ligand which binds directly to a nucleosolic protein which comprises:
a. culturing the compound with a eukaryotic cell expressing said nucleosolic protein:
b. exposing the eukaryotic cell to a detectably labelled compound which is known to bind to the nucleosolic protein;
c. separating the nucleus from said eukaryotic cell, which nucleus comprises a liquid nucleosol fraction and a particulate membrane fraction; and
d. determining through the presence of labelled material in the nucleosol fraction whether the compound or the ligand it produces or the labelled compound is bound to the nucleosolic protein.

3. The methods of claims 1 and 2 wherein the nucleosolic protein is a nuclear receptor.

4. The methods of claim 3 wherein the level of expression of the receptor is about 25,000 to about 100,000 receptors per cell.

5. The methods of claim 3 wherein the nuclear receptor is a retinoic acid receptor.

6. The methods of claim 5 wherein the retinoic acid receptor is the RXR receptor.

7. The methods of claims 1 and 2 wherein the nucleosolic protein has an affinity for the ligand of at least about 10⁻⁸ molar.

8. The methods of claims 1 and 2 wherein the detectable label is an isotopic label.

9. The methods of claims 1 and 2 wherein the eukaryotic cell is a COS cell transformed with an expression vector comprising the SV40 origin of replication.

10. Use of a method as claimed in claims 1 and 3 to 9 for isolating the ligand of a nucleosolic protein.

11. Use of a method as claimed in claims 2 to 9 for determining whether a compound produces a ligand which binds directly to a nucleosolic protein.


SEQUENCE LISTING

(1) GENERAL INFORMATION:

(i) APPLICANT:
(A) NAME: F. HOFFMANN-LA ROCHE AG
(B) STREET: Grenzacherstrasse 124
(C) CITY: Basle
(D) STATE: BS
(E) COUNTRY: Switzerland
(F) POSTAL CODE (ZIP): CH-4002
(G) TELEPHONE: 061 - 688 42 56
(H) TELEFAX: 061 - 688 13 95
(I) TELEX: 962292/965542 hlr ch

(ii) TITLE OF INVENTION: Methods for determining and isolating
compounds which bind directly to nucleosolic proteins

(iii) NUMBER OF SEQUENCES: 2
(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 1:

GATCTGCTGT CACAGGTCAC AGGTCACAGG TCACAGTTCA
40

(2) INFORMATION FOR SEQ ID NO: 2:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 2:

GATCTGAACT GTGACCTGTG ACCTGTGACC TGTGACAGCA
40